# EUROPEAN PATENT SPECIFICATION

(11) **EP 0 661 990 B1**
(45) Date of publication and mention of the grant of the patent: **10.04.2002**
(21) Application number: 93920354.3
(22) Date of filing: 26.08.1993
(51) Int. Cl.: A61K 38/30, A61K 38/43, C07K 7/00

(54) **METHOD FOR SYSTEMIC TREATMENT OF CATABOLIC CONDITIONS AND SYSTEMIC TISSUE INJURY**
VERFAHREN ZUR SYSTEMISCHEN BEHANDLUNG KATABOLISCHER ZUSTÄNDE UND SYSTEMISCHER GEWEBESCHÄDIGUNG
PROCEDE DE TRAITEMENT SYSTEMIQUE D'ETATS CATABOLIQUES ET DE LESIONS TISSULAIRES SYSTEMIQUES

(30) Priority: 26.08.1992 US 935890; 03.12.1992 US 984936
(43) Date of publication of application: 12.07.1995
(73) Proprietor: CELTRIX PHARMACEUTICALS, INC., Santa Clara, CA 95054-1815 (US)
(72) Inventor: SOMMER, Andreas, Concord, CA 94518 (US); MAACK, Christopher, A., El Cerrito, CA 94530 (US)
(74) Representative: Goldin, Douglas Michael
(86) International application number: US9308025
(87) International publication number: WO9404030

(56) References cited:
- WO-A-92/13556
- US-A- 5 187 151
- JOURNAL OF ORTHOPAEDIC RESEARCH, vol. 10,no. 1, January 1992 pages 14-22, TESCH G.H. ET AL.
- JOURNAL OF ENDOCRINOLOGY, vol. 135, 1992 pages 135-142, CWYFAN HUGHES S.C. ET AL
- E.M. SPENCER, "Modern Concepts of Insulin-Like Growth Factors", published 1991 by Elsevier (NY), pages 715-728, see entire document.

## Description

### Technical Field

This invention relates to metabolic disorders and aids in tissue repair. This invention concerns catabolic conditions in individuals with a loss of tissue protein. This invention aids in tissue repair of burns, peptic ulcers, and traumatic injury. The invention comprises a complex comprising an insulin-like growth factor (IGF) and insulin-like growth factor binding protein-3 (IGFBP-3) for manufacturing a medicament for systemic (but not subcutaneous bolus) administration.

### Background Art

Growth factors are polypeptides which stimulate a wide variety of biological responses (e.g., DNA synthesis, cell division, expression of specific genes, etc.) in a defined population of target cells. A variety of growth factors have been identified including transforming growth factor-β₁ (TGF-β₁), TGF-β₂, TGF-β₃, epidermal growth factor (EGF), platelet-derived growth factor (PDGF), fibroblast growth factor (FGF), insulin-like growth factor-I (IGF-I), and IGF-II.

IGF-I and IGF-II are related in amino acid sequence and structure, with each polypeptide having a molecular weight of approximately 7500 daltons. IGF-I mediates the major effects of growth hormone and thus is the primary mediator of skeletal growth after birth. IGF-I has also been implicated in the actions of various other growth factors, since treatment of cells with such growth factors leads to increased production of IGF-I. In contrast, IGF-II is believed to have a major role in fetal growth. Both IGF-I and IGF-II have insulin-like activity (hence the name) and are mitogenic (stimulating cell division) for the cells in muscle, skeletal tissue and a wide variety of other tissues.

Unlike most growth factors, the IGFs are present in substantial quantity in the circulation, but only a very small fraction of this IGF is free in the circulation or in other body fluids. Most circulating IGF-I is bound to an IGF-binding protein called IGFBP-3. IGF-I may be measured in blood serum to diagnose abnormal growth-related conditions, e.g., pituitary gigantism, acromegaly, dwarfism, various growth hormone deficiencies, etc. Although IGF-I is produced in many tissues, most circulating IGF-I is believed to be synthesized in the liver.

Almost all IGF circulates in a non-covalently associated ternary complex composed of IGF-I or -II, an IGF specific binding protein termed IGFBP-3, and a larger protein termed the Acid Labile Subunit (ALS). This ternary complex is composed of equimolar amounts of each of the three components. The ALS has no direct IGF binding activity and appears to bind only a preformed IGF/IGFBP-3 complex. The ternary complex of IGF + IGFBP-3 + ALS has a molecular weight of approximately 150,000 daltons. This ternary complex is alleged to function in the circulation "as a reservoir and a buffer for IGF-I and IGF-II preventing rapid changes of free IGF." See, Blum, W.F., et al., "Plasma IGFBP-3 Levels as Clinical Indicators", In Modern Concepts in Insulin-Like Growth Factors, E. M. Spencer, ed., Elsevier, New York, pages 381-393, 1991.

Nearly all of the IGF-I, IGF-II and IGFBP-3 in the circulation are in complexes, so very little free IGF or IGFBP-3 is detectable. Moreover, a high level of free IGF in plasma is undesirable. It would lead to serious hypoglycemia because IGF has insulin-like effects on circulating glucose levels. In contrast to the IGFs and IGFBP-3, there is a substantial pool of free ALS in plasma which assures that IGF/IGFBP-3 complex entering the circulation immediately forms a ternary complex.

IGFBP-3 is the most abundant IGF binding protein in the circulation, but at least five other distinct IGF binding proteins have been identified in various tissues and body fluids. Although these proteins bind IGFs, they each originate from separate genes and they have distinct amino acid sequences. Thus, the binding proteins are not merely analogs of a common precursor. Unlike IGFBP-3, the other IGFBPs in the circulation are not saturated with IGFs. None of the IGF binding proteins other than IGFBP-3 can form the 150 KD circulating ternary complex.

IGF-I and IGFBP-3 may be purified from natural sources or produced by recombinant means. For instance, IGF-I has been purified from human serum for a number of years. See, Rinderknecht, E.W., et al., Proc Natl Acad Sci (USA) 73, 2365-2369, 1976. Recombinant IGF-I processes are shown in EPA 0,128,733, published in December of 1984. IGFBP-3 may be purified from natural sources using a process such as that shown in Baxter et al., "Growth Hormone-Dependent Insulin-Like Growth Factors (IGF) Binding Protein from Human Plasma Differs from Other Human IGF Binding Proteins", Biochem Biophys Res Comm 139, 1256-1261, 1986. IGFBP-3 may be synthesized by recombinant organisms as discussed in Sommer, A. S., et al., In Modern Concepts of Insulin-Like Growth Factors, E. M. Spencer, ed., Elsevier, New York, pp. 715-728, 1991. This recombinant IGFBP-3 binds IGF-I in a 1:1 molar ratio. The topical administration of the IGF-I/IGFBP-3 complex to rat and pig wounds was significantly more effective than IGF-I alone. Sommer et al., ibid. Intravenous administration of the complex to hypophysectomized rats "substantially prevents the hypoglycemic effects of IGF-I" administered alone. Sommer et al., ibid.

U.S. Patent No. 5,128,320 issued to Hahn et al. discloses a method for restoring weight gain and lean body mass in a mammal afflicted with glucocorticoid excess, which is endogenously or exogenously produced.

U.S. Patent No. 5,126,324 issued to Clark et al. discloses a method for enhancing growth in a mammal by administration a combination of IGF-I and growth hormone (GH). Clark et al. mention that the technique is particularly useful in animals no longer responsive to GH alone.

### Catabolic States

Many patients have illnesses and other abnormalities that lead to debilitating catabolic states. Specifically, when people are chronically nutritionally deprived and/or expend an inordinate amount of calories (as in chronic obstructive pulmonary disease, or COPD), they burn body fat and protein. The protein comes from sacrificing needed enzymes and muscles. When protein is used for energy, the body excretes nitrogen. If patients who excrete nitrogen have a negligible intake of nitrogen-containing nutrients, such patients excrete more nitrogen than is ingested and therefore have a negative nitrogen balance. Catabolic conditions in which this occurs include, but are not limited to, chronic obstructive pulmonary disease, gastrointestinal tract resections or disorders, illnesses requiring corticosteroid therapy, diabetes, trauma, pneumonia, heart failure, stroke, cancer cachexia, and AIDS cachexia. Catabolism is associated with these illnesses and is characterized by negative nitrogen balance or protein wasting. Severe loss of body protein substantially increases chances for dying and/or prolonged hospitalization and major medical expenses.

An additional group of patients who are at risk of negative nitrogen balance are patients in hospitals or nursing homes who are convalescing from acute illnesses. Every year, several million elderly patients are hospitalized with problems running the gamut from pneumonia, to heart failure, broken bones, strokes, and cachexia due to tumors. Many of these patients cannot leave the hospital because they are too debilitated to ingest adequate nutrients to restore muscle mass and strength that would enable them to manage outside a hospital environment. Often, heroic measures such as total parenteral nutrition (TPN) are attempted to improve the prognosis for severely wasted patients. But in many instances, even TPN is not effective (The Veterans Affairs TPN Cooperative Study Group, N Engl J Med 325:525-32, 1991).

Increasing the circulatory level of growth hormone (Jiang, Z.-M., et al., Ann Surg 210, 513-525, 1989) or IGF-I (Pape, G. S., et. al., Chest 99, 1495-1500, 1991) has been shown to be effective in restoring or increasing positive nitrogen balance and in maintaining muscle mass in a variety of human and animal model studies. However, patients undergoing these treatments must be carefully monitored in order to avoid significant side effects.

### Wound Healing Following Surgery or Trauma

Numerous investigators have reported higher levels of IGF in injured tissue (Spencer et al. Growth Factors and Other Aspects of Wound Healing (1988), pp. 103-16 (surgical wound and implanted wound canister); Jennische et al. Exp. Mol. Pathol. (1987) 47:193-201 (freeze-thaw injury); Gartner et al. J. Surg. Res. (1992) 52:389-94 (implanted sponge and controlled length scar models; high levels of expression of IGF-1 and IGF-2 mRNA in wounds after 1 day). Skottner et al. (Acta Pediatr. Scand. [Suppl.] (1988) 347:110-12) reported that local application of IGF-1 to a crush lesion of rat sciatic nerve significantly increased nerve regeneration which could be inhibited by anti-IGF-1 antibodies.

Drop et al. (W089/08667), after disclosing an amino acid sequence for IGFBP-1 and its recombinant production, briefly mention that this binding protein could be administered with a variety of growth factors, including IGF-1 and IGF-2, either locally or systemically. Drop et al. further proposed that such formulations could be used in healing wounds and treating osteoporosis.

### Wound Healing in Burn Patients

In patients with severe burns, the IGF-1 level is low (Coates et al., Burns (1981) 7:425; Cunningham et al., Proc. Amer. Burn Assoc. (1986) 18:128). Moller et al. (Burns (1991) 17:279-81) correlated the decreased IGF-1 level with the surface area of the burn and proposed that the low level may be due to IGF diffusion from the burned skin. The reduced IGF level persisted for about 3-4 weeks after large burns. Moller et al. proposed that the reduced "IGF-1 concentration may contribute to the reduced wound healing." (page 280)

Strock et al. (Surgery (1990) 108:161-64) studied the effects of IGF administration on Sprague-Dawley rats which were given a full-thickness burn over one half of the body surface area. After the burn, osmotic pumps administering 1000 µg/day of IGF were implanted in the rats. Circulating IGF levels decreased markedly after the burn before the pumps were implanted. Body weight increased significantly for IGF-treated burned rats, particularly in comparison with the burned rats not receiving IGF, even though control rats were fed the same amount as IGF-treated rats. The increase in body weight was interpreted as "a direct anabolic effect of IGF-I in the postinjury state." (page 163)

### Wound Healing with Glucocorticoid Therapy

In EP 0 434 625 A2 publication filed 13 December 1990, Goldberg disclosed the administration of IGF-1 either alone to counteract the effect of endogenous corticosteroids or the administration of IGF-1/glucocorticoid cocktails, wherein IGF-1 would blunt the deleterious effect of steroids on cartilage and wound repair, particularly in osteoarthritis.

The prior art has not suggested that systemic administration of the combination of IGF and IGFBP could aid in burn healing, in recovery from surgical wounds, or in wound healing impaired by steroid administration.

### Disclosure of Invention

In accordance with the present invention, there is provided a use of a complex comprising an insulin-like growth factor (IGF) and insulin-like growth factor binding protein-3 (IGFBP-3) in the manufacture of a systemically administered medicament, other than one adapted for subcutaneous bolus administration for treating a catabolic condition.

The IGF used in the complex may be provided as IGF-I. The IGF and IGFBP may be present in equimolar amounts.

Both IGF and IGFBP-3 can be human proteins obtained from recombinant sources.

The complex of IGF and IGFBP-3 is administered by subcutaneous injection, and may provide treatment of a protein wasting disease with a complex of IGF and IGFBP-3.

The individual to whom the complex is administered may be a mammalian or avian individual.

The IGF/IGFBP-3 complex may be administered in an amount sufficient to result in a positive nitrogen balance. The amount of IGF/IGFBP-3 complex administered may be at least about 0.05 to 10 mg of IGF/kg/day.

The present invention may allow a method for enhancing tissue repair in an individual who is about to undergo, is undergoing or has just undergone surgery. The method provides for systemic administration to the individual of a therapeutic composition including insulin-like growth factor (IGF) and insulin-like growth factor binding protein (IGFBP) in quantities sufficient to enhance tissue repair and accelerate healing.

The present invention can also provide a method for treating a burned individual to enhance healing of the burn. The method provides for systemic administration to the individual of a therapeutic composition including insulin-like growth factor (IGF) and insulin-like growth factor binding protein (IGFBP) in a quantity sufficient to enhance healing of the individual's burn.

The present invention can also be used in a method for treating an individual who has experienced traumatic injuries to enhance repair of hard and soft tissue. The method provides for systemic administration to the individual of a therapeutic composition including insulin-like growth factor (IGF) and insulin-like growth factor binding protein (IGFBP) in a quantity sufficient to enhance healing of the individual's injuries.

The present invention may find use in a method for treating an individual who has a peptic ulcer to enhance healing of the ulcer. The method provides for systemic administration to the individual of a complex including insulin-like growth factor (IGF) and insulin-like growth factor binding protein (IGFBP) in a quantity sufficient to enhance healing of the individual's peptic ulcer.

In accordance with another embodiment of the present invention, there is thus provided a use of IGF and IGFBP according to claim 9.

The individual undergoing surgery may be having a tumor resected, having an organ or body part removed or replaced, may be receiving glucocorticoid therapy or is diabetic.

The mode of systemic administration can be parenteral or gastrointestinal. Parenteral forms of administration include subcutaneous, intravenous intraperitoneal and intramuscular injection.

The IGF may be IGF-I, such as recombinant human IGF-I.

The IGFBP may be IGFBP-3, such as recombinant human IGFBP-3.

The individual to whom the therapeutic composition is administered can be a mammal.

The IGF/IGFBP composition may be administered in an amount of about 0.01 to 5 mg of IGF/kg/day bound to an approximately equimolar amount of IGFBP-3.

While not wishing to be bound by any particular theory, the Inventors propose that the administered complex of IGF and IGFBP-3 treats catabolic states by producing the gradual release of free IGF in somewhat elevated levels. The added IGF is believed to promote cell anabolism and thereby alleviate the continuing loss of muscle mass and strength in catabolic conditions and promote restoration of muscle mass and strength. Moreover, the Inventors propose that the systemically administered IGF and IGFBP composition causes systemic healing by first raising the blood level of IGF/IGFBP. The IGF is then carried to the regenerating tissues via the circulation.

### Brief Description of the Drawing

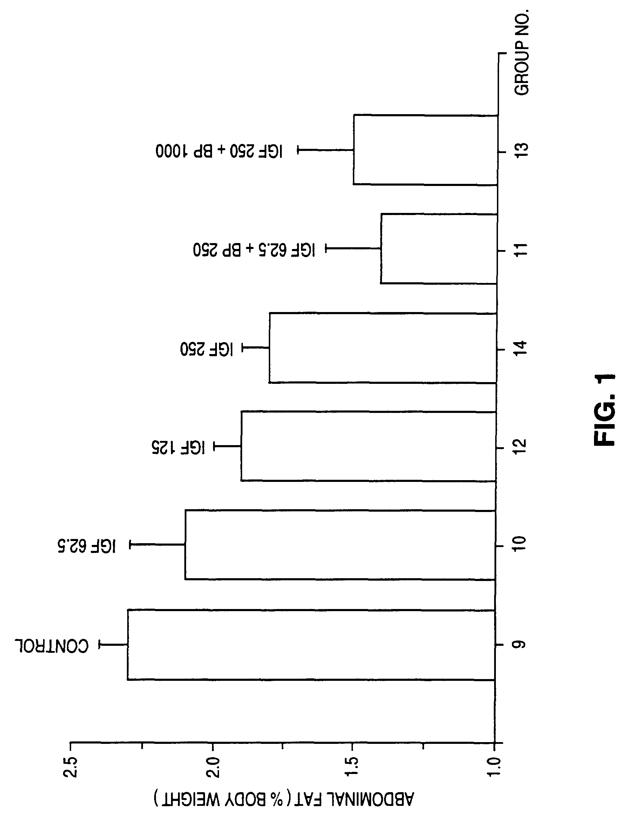

Figure 1 is a bar graph depicting the results of various treatments (including the inventive treatment) on broiler chickens.

### Modes For Carrying Out the Invention

Definitions:
As used herein, "nitrogen balance" is defined as the comparison of nitrogen intake with nitrogen excretion. A positive nitrogen balance is achieved when a patient takes in more nitrogen than is excreted. A negative nitrogen balance occurs when there is tissue breakdown and the individual excretes more nitrogen than is taken in.
A "catabolic condition" is one in which an individual has a net breakdown of tissue. This contrasts with an anabolic state in which an individual has a net increase in body tissue, such as increasing muscle mass.
"Individuals" are defined as humans and mammal and avian farm animals, sport animals and pets. Farm animals include, but are not limited to, cows, hogs, sheep, chicken, turkeys, ducks and geese. Sport animals include, but are not limited to, dogs and horses. The category pets includes, but is not limited to, cats, dogs, and birds.
"Insulin-like growth factor (IGF)" comprises a family of factors, including but not limited to IGF-I and IGF-II. IGF is a polypeptide having a molecular weight of about 7500 daltons. IGF may be obtained from natural sources or prepared by recombinant means.
"Insulin-like growth factor binding protein (IGFBP)" comprises a family of binding proteins, including but not limited to IGFBP-1, IGFBP-2, IGFBP-3, IGFBP-4, IGFBP-5 and IGFBP-6. IGFBP may be obtained from natural sources or prepared by recombinant means. At least one form of IGFBP (for example, IGFBP-3) complexes with IGF and with a third molecule known as ALS.
A "therapeutic composition" as used herein is defined as comprising IGF complexed with its binding protein IGFBP-3. The therapeutic composition may also contain excipients such as water, minerals and carriers such as protein.
"Systemic administration" is any administration to an animal as a whole. It does not include local application of the complex directly onto a wound. Systemic administration includes parenteral and gastrointestinal and nasal routes. The parenteral route includes a wide variety of administrations, including intravenous, subcutaneous (not subcutaneous bolus), intraperitoneal and intramuscular routes. The gastrointestinal route includes oral, enteral and rectal administration.

One use of the present invention contemplates treating and alleviating the catabolic state associated with a variety of diseases by administering a complex of IGF and IGFBP-3. Another use of the present invention contemplates enhancing systemic tissue repair, nitrogen balance and immune function in a variety of conditions, including, but not limited to, individuals about to undergo, undergoing or having undergone surgery, individuals with burns; individuals with traumatic injuries; and individuals with peptic ulcers, by administering a therapeutic composition of IGF/IGFBP.

Individuals undergoing surgery often have difficulty maintaining a positive nitrogen balance. This can interfere with the body's healing processes. If insufficient nutrients are available or inefficiently marshaled, healing is inefficient, and hospital stays may be prolonged.

Some individuals have more difficulty healing after surgery, particularly individuals with tumors, individuals receiving glucocorticoids and diabetics.

Individuals with cancer may be wasted and in negative nutritional balance prior to surgery. Without extra supportive medical care, healing after tumor resection may be slow or even incomplete. Administration of the IGF/IGFBP complex aids healing.

Diabetics who undergo surgery often heal slowly or inadequately. During surgery, stress causes high plasma levels of cortisol, which antagonizes the effect of insulin. Therefore, insulin-requiring diabetics require more insulin during surgery. Typically, the physician gives such the diabetic 1/3 to 1/2 of a normal daily insulin dose before surgery. As the diabetic recovers from surgery, the insulin dose is repeated. In the meantime, 5% glucose solution is slowly infused and adjusted to maintain blood sugar levels. Administration of IGF/IGFBP beginning before surgery would increase the blood level of IGF and should aid in overcoming the effects of cortisol. IGF/IGFBP can enhance healing in the post-surgical diabetic, both by its direct effect on the healing process and by its insulin-like effects on glucose utilization.

Individuals who have been on glucocorticoid therapy prior to surgery can benefit from the administration of the IGF/IGFBP composition. Glucocorticoids include, but are not limited to, cortisol, prednisolone, triamcinolone, dexamethasone, beclomethasone, betamethasone, and budesonide. Cortisol and its analogs suppress the inflammatory response and also suppress fibroblast proliferation and collagen deposition. These actions impair wound healing. Because of these actions, peptic ulcers and capillary fragility are occasional side effects of steroid administration. Administration of the IGF/IGFBP composition can overcome these deleterious effects of glucocorticoids.

After prolonged administration, abrupt withdrawal of steroid can cause a life-threatening condition because normal adrenal function has been suppressed and is insufficient. Administration of the IGF/IGFBP complex helps counteract the negative effects of steroids on individuals undergoing surgery and permits the physician to gradually withdraw steroid therapy. In such cases, the IGF/IGFBP composition also can enhance wound healing.

Burn sufferers can benefit from administration of the IGF/IGFBP complexes. Burn patients have wounds to heal. The complex delivers IGF in a gradual manner to aid healing without causing hypoglycemia.

Peptic ulcers occur in individuals receiving some anti-inflammatory drugs and in numerous other individuals. Peptic ulcers are related in part to acid secretion; however, most individuals secrete stomach acid. but few have ulcers. Peptic ulcers probably arise when the gastrointestinal mucosal lining does not replace itself quite rapidly enough. Acid erosion then progresses through the mucosa into the submucosal layer and even into the muscular layer. Peptic ulcers may occur in the stomach (gastric) or in the upper intestine (duodenal). In the same way that IGF/IGFBP encourages wound healing in other areas, IGF/IGFBP can help heal peptic ulcers.

Nearly all IGF-I or IGF-II complex with IGFBP-3 and IGF/IGFBP-3 normally circulate in the form of a complex in humans and other mammals and avians. This complex associates with a third protein (ALS), which is present in excess over the concentration of IGF and IGFBP-3. Therefore, ALS is found both associated with the IGF/IGFBP-3 complex and in the free form. The resultant ternary complex has a size of about 150 kD. Administration of the complex of IGF and IGFBP-3, either from natural or recombinant sources, as a preformed complex results in the formation of the ternary complex with the normally excess ALS. This type of treatment appears to produce a long term increase in the level of circulating IGF, which is gradually released from the ternary complex. This mode of administration avoids the detrimental side effects associated with administration of free IGF-I, e.g., hypoglycemia, suppression of growth hormone and ALS production, and release of endogenous IGF-II since administered exogenous free IGF-I replaces endogenous IGF-II in normally circulating IGF-II/IGFBP-3 complexes.

The formulation, method of administration and dosage will depend upon the disorder to be treated, and the medical history of the patient. These factors are readily determinable in the course of therapy. Suitable patients with a catabolic disorder, wasting disease, burns, trauma, ulcers or a need for surgery can be identified by medical history, physical findings and laboratory tests. The medical history may reveal such facts as recent weight loss, decreasing ability to perform normal functions of life, and illness or surgery. Patients may have physical findings such as low weight for their age and height, low abdominal or triceps skin-fold thickness, low hand-grip strength, low arm-muscle circumference and poor pulmonary function due to weakened respiratory muscles. Indicative laboratory results include low levels of serum prealbumin and albumin and abnormally high levels of nitrogenous wastes in the urine. When the amount of excreted nitrogen exceeds the nitrogen intake, the patient is in negative nitrogen balance.

In accordance with the use of the present invention, the formulation comprises a complex of IGF and IGFBP-3. Preferably, the IGF is IGF-I, although IGF-II may be useful. In another embodiment, IGF can be a mixture of IGF-I and IGF-II. In such a mixture, the ratio of IGF-I to IGF-II ranges from .01 to 99.

IGFBP can be any of IGFBP-1, -2, -3, -4, -5 or -6. IGFBP also can be a mixture of any combination of the six IGFBP's. Such a mixture would take advantage of the different binding affinities for IGF-I and IGF-II, ability of some IGFBP's to bind to cell surfaces, and different half-lives.

The molecular structure of IGFBP-1 was disclosed by Brewer et al., Biochem. Biophys. Res. Comm. (1988) 152(3):1289-1297 and by Drop et al. in PCT Publication No. WO 89/98667, published on September 21, 1989. Human IGFBP-1 has 234 amino acids and a molecular weight of about 28 kd. In combination with IGF-I, IGFBP-1 seems to stimulate thymidine incorporation into cellular DNA. Busby et al., J. Biol. Chem. (1988) 263:14203-10; Elgin et al., Proc. Natl. Acad. Sci. USA (1987) 84:3254-58. Drop et al. (ibid.) suggested that IGFBP-1 would be useful in tissue repair because of its potentiation of growth of connective tissue and muscle cells.

IGFBP-2 comprises 289 amino acids (human) and has a molecular weight of 36 kd under nonreducing conditions. The amino acid sequence of human IGFBP-2 was determined from cDNA clones isolated from a human fetal liver library by Binkert et al. EMBO J. (1989) 8:2493-2502. IGFBP-2 also may bind to cell surfaces. IGFBP-2 has a preference for IGF-II, and thus is preferred in formulations comprising IGF-II.

Preferably the IGFBP in the IGF/IGFBP complex is IGFBP-3. Native and recombinant IGFBP-3, as well as some N-terminal and C-terminal fragments, bind IGF-I and IGF-II. Human IGFBP-3 comprises 264 amino acids and has three potential N-linked glycosylation sites. IGFBP-3 is the major IGFBP in blood.

Nearly all IGF-I or IGF-II in blood is bound to IGFBP-3, and IGF/IGFBP-3 normally circulates in the form of a complex in humans and other mammals. This complex associates with a third protein (ALS), which is present in excess over the normal concentrations of IGF and IGFBP-3. Therefore, ALS is found both associated with the IGF/IGFBP-3 complex and in the free form. The resultant ternary complex has a size of about 150 kD. Administration of the complex of IGF and IGFBP-3, either obtained from natural or recombinant sources, results in the formation of the ternary complex with the normally excess ALS. This type of treatment appears to produce a long term increase in the level of circulating IGF, which is gradually released from the ternary or binary complex. This mode of administration avoids the detrimental side effects associated with administration of free IGF-I (e.g., hypoglycemia, suppression of growth hormone and ALS production, and release of endogenous IGF-II from endogenous IGFBP-3 since administered free IGF-I replaces endogenous IGF-II in normally circulating IGF-II/IGFBP-3 complexes).

IGFBP-4 and IGFBP-6 are glycosylated proteins which are widely distributed in the body. The primary structure of IGFBP-4 was reported by Shimasaki et al. Mol. Endocrinol. (1990) 4:1451-1458. IGFBP-6, whose cDNA has been isolated by Shimasaki et al. (Mol. Endocrinol. (1991) 4:938-48), has a much greater affinity for IGF-II than for IGF-I.

IGFBP-5 is a 252 amino acid binding protein which is not glycosylated. Shimasaki et al. (J. Biol. Chem. (1991) 266:10646-53) cloned human IGFBP-5 cDNA from a human placenta library.

Depending on the binding, metabolic and pharmacokinetic characteristics required in the IGF/IGFBP complex formulation, these binding proteins can be added to the complex formulation in various proportions. These IGFBP's can be combined in a wide variety of ratios with IGF-I and/or IGF-II.

Because IGF and IGFBP-3 naturally complex in a 1:1 molar ratio, a composition of equimolar amounts of IGF and IGFBP-3 is preferred. The product can be formulated with IGF:IGFBP-3 molar ratios ranging from 0.5 to 1.5. More preferably, the molar ratio is 0.9 to 1.3; and most preferably, the product is formulated with approximately a 1:1 molar ratio.

In accordance with the use of the present invention, IGF and IGFBP-3 are human proteins obtained from natural or recombinant sources. Most preferably, IGF and IGFBP-3 are human IGF-I and IGFBP-3 made by recombinant means and designated rhIGF-I and rhIGFBP-3, respectively. rhIGFBP-3 may be in glycosylated or non-glycosolated form. *E. coli* is a source of the non-glycosolated IGFBP-3. Glycosylated IGFBP-3 may be obtained from CHO-cells.

The use of the present invention provides for formulating the complex in modes which are readily apparent to those skilled in the art. Preferably, the IGF and IGFBP-3 are complexed prior to administration to the treated individual. Preferably, the complex is formed by mixing approximately equimolar amounts of IGF-I and IGFBP-3 dissolved in physiologically compatible carriers such as normal saline solution or phosphate buffered saline solution. Most preferably, a concentrated solution of rhIGF-I and a concentrated solution of IGFBP-3 are mixed together for a sufficient time to form an equimolar complex.

Depending on the mode of administration, compositions of the complex may be in the form of solid, semi-solid or liquid dosage preparations, such as for example, tablets, pills, powders, capsules, liquids, suspensions or the like. Physiologically compatible carriers include intravenous solutions, such as normal saline, serum albumin, 5% dextrose, plasma preparations, other protein-containing solutions and TPN solutions. The preferred carrier for parenteral administration of the complex is a sterile, isotonic aqueous solution, such as normal saline or 5% dextrose. Alternatively, a solution of the complex may be placed into an implant, such as an osmotic pump, for the slow release of the complex over an extended period of time. Alternatively, the complex may be provided in sustained release carrier formulations such as semi-permeable polymer carriers in the form of suppositories or microcapsules. See, for instance, U.S. Patent No. 3,773,919 for Microcapsular Sustained Release Matrices Including Polylactides; Sidmon et al., Biopolymers 22 (1), 547-556 (1983) for copolymers of L-glutamic acid and γ-ethyl-L-glutamate; Langer et al., J Biomed Res 15, 167-277 (1981) for poly(2-hydroxyethylmethacrylate) or the like.

The mode of administration delivers the complex to the individual in a safe, physiologically effective manner. The complex may be given by intranasal, subcutaneous, intravenous, intraperitoneal, or other conventional routes of administration. Preferably, the complex is injected subcutaneously, (but not by subcutaneous bolus), intravenously or intramuscularly. Most preferably, the complex is administered by subcutaneous injection. By subcutaneous injection, the complex appears not to be toxic or mitogenic at the injection site. In another preferred mode of administration, the complex is administered by continuous intravenous infusion in combination with TPN solutions.

The dose of complex to be administered can be readily determined by those skilled in the art, based on the usual patient symptoms discussed above. Preferably, when the complex is administered to humans daily, the dosage of complex is at least about 0.05 mg IGF/kg of body weight/day, complexed to an equimolar amount of IGFBP-3. More preferably, the daily dosage of the complex for humans is at least 0.1 mg IGF/kg/day, complexed to an equimolar amount of IGFBP-3. If daily dosages in excess of about 0.5 mg IGF/kg must be given, the dosage may be divided and injected subcutaneously at two or more sites.

If the IGF/IGFBP-3 complex were administered to humans twice a week, each dose of complex is preferably at least about 0.1 mg IGF/kg of body weight, complexed to an equimolar amount of IGFBP-3. More preferably, for twice weekly administration, the dose of the complex is at least 0.5 mg IGF/kg, complexed to an equimolar amount of IGFBP-3. There is no known upper limit of dosage; however, it is preferable that a single dose not exceed 10 mg IGF/kg of body weight, when the IGF is complexed to an equimolar amount of IGFBP-3. These doses of IGF/IGFBP-3 complex are not expected to cause significant hypoglycemia since IGFBP-3 slows the IGF binding to cellular insulin receptors.

Preferably, the malnourished patient or patient about to undergo surgery is started with a relatively low dose of the complex, such as 0.05 mg of IGF-I complexed with an equimolar amount of IGFBP-3/kg of body weight/day. The various factors given above should be monitored to determine if there is improvement. Preferably, the patient's nitrogen balance becomes positive. These include, but are not limited to, systemic IGF-I levels, weight, hand-grip strength, arm-muscle circumference, serum prealbumin, serum glucose, serum albumin, and the appearance of wounds, burns or ulcers. If the patient improves with the low dose, the low dose preferably should be continued until the patient's wasting is ameliorated or nutritional status is adequately improved, as indicated by the physical findings and laboratory results described above. For example, hand-grip strength and/or pulmonary function should improve; the surgical wound or burn should be healing satisfactorily. Such improvement may be evident in two to three weeks.

If the patient's nitrogen balance does not become positive after the low dose of the complex, the dose preferably should be increased gradually until the nitrogen balance becomes positive.

In the hospital, intravenous infusions and subcutaneous injections of the IGF/IGFBP complex are preferred. In the clinic or doctor's office, subcutaneous injections frequently are preferred.

Somewhat higher per kilogram doses are needed for small animals receiving the IGF/IGFBP-3 complex. For example, a bird may be dosed twice a week with about .05 to 1.0 mg of IGF/kg of body weight.

The invention has been disclosed by direct description. The following are examples showing the efficacy of the method in increasing muscle mass, lean body mass, and systemic tissue repair. The examples are only examples and should not be taken in any way as limiting to the scope of the method.

### Examples

### Example 1

This experiment shows the effect of the complex of IGF-I and IGFBP-3 upon lean body mass production as opposed to fat production. In this experiment, human recombinant IGF-I and IGFBP-3 were used. The rhIGF-I (Ciba-Geigy) was synthesized in yeast and provided in sterile water and stored at -70° C. The rhIGFBP-3 (Celtrix Laboratories, Inc., Santa Clara, CA) was synthesized by *E. coli* and was not glycosylated; IGFBP-3 was dissolved in phosphate-buffered saline and stored at -70° C until use. Prior to administration, the proteins were thawed, and sufficient amounts of IGF-I and IGFBP-3 were mixed to provide equimolar amounts of the two proteins. Groups of growing broiler chickens were treated with various doses of free IGF-I or IGF-I/IGFBP-3 complex. A control group of chickens was treated only with placebo or the vehicle. All treatments were administered by subcutaneous injection three times a day. The doses were administered for two weeks between days 25 and 39 of life, when the chickens were still growing.

The overall growth rate of the animals was not affected by treatment with free IGF-I or IGF-I/IGFBP-3 complex at any dose level. However, the effect on the accumulation of fat in the abdominal fat pad, as illustrated in Figure 1, was quite striking. Treatment with free IGF-I at daily doses of 62.5, 125 or 250 µg/kg (Groups 10, 12 and 14, respectively) resulted in a progressive decrease in abdominal fat pad weight as a percentage of total body weight compared to control animals treated with vehicle (Group 9). Surprisingly, when IGF-I was administered with IGFBP-3 in the IGF-I/IGFBP-3 complex, a much larger effect was obtained. The lowest daily dose of IGF-I/IGFBP-3 complex (62.5 µg IGF-I/kg + 250 µg IGFBP-3/kg, Group 11) resulted in the largest decrease in abdominal fat pad weight. This fat reduction amounted to a decrease of 40% in the weight of fat pads of treated animals compared to those of control animals. This decrease in fat pad weight could not be equalled by even a four-fold higher dose of free IGF-I. This may be the maximum fat pad reduction obtainable, as the higher dose of IGF-I/IGFBP-3 complex did not lead to further reduction.

Because IGF is not known to have a preferential effect on abdominal fat pads over other body fat, we expected that the decreased deposition of fat in the abdominal fat pad reflects fat reduction in the rest of the body. Since overall body weight gain was not affected by these treatments, and no obvious edema was observed, we expect that body fat reductions were balanced by increases in lean body mass in treated animals. And this was confirmed by the experiment in Example 2.

### Example 2

This example shows the use of free IGF-I and the IGF-I/IGFBP-3 complex on female rats with ovariectomy-induced osteoporosis. Both rhIGF-I and rhIGFBP-3 were obtained as mentioned above. This experiment demonstrates the ability of the IGF-I/IGFBP-3 complex to increase muscle mass and lower fat mass.

In this example, young female rats of 90-100 g body weight were ovariectomized by the dorsal route and were divided into six groups of eight animals each. An additional group consisted of eight intact, age-matched sham operated control rats. Six weeks after ovariectomy, treatment of the animals was started as follows:
- Group 1:: Sham Operated Controls; Vehicle
- Group 2:: Ovariectomized Controls; Vehicle
- Group 3:: Ovariectomized; 2.5 mg/kg IGF-I complexed to 9.5 mg/kg IGFBP-3
- Group 4:: Ovariectomized; 0.25 mg/kg IGF-I complexed to 0.95 mg/kg IGFBP-3
- Group 5:: Ovariectomized; 0.025 mg/kg IGF-I complexed to 0.095 mg/kg IGFBP-3
- Group 6:: Ovariectomized; 2.5 mg/kg IGF-I
- Group 7:: Ovariectomized; 0.25 mg/kg IGF-I

The complex was formed by mixing equimolar amounts of IGFBP-3 (dissolved in phosphate buffered saline (PBS), pH 6.0) and IGF-I (dissolved in 10 Mm sodium acetate, pH 5.5) in the minimum volume feasible, and incubating the mixture overnight at 4°C. The complex was then diluted with PBS, pH 6.0, containing 0.1% rat serum albumin. The solutions were divided into aliquots containing the amount of material needed for one day, and stored at -70°C until needed. The controls received the dilution buffer.

The animals were treated for 22 days. The test substances were administered six times per week by one daily subcutaneous injection. One day before treatment was started and on the 17th day of treatment, 20 mg/kg of calcein was given by intraperitoneal injection. Calcein is a tetracycline which deposits in growing bone and is used to estimate the amount of bone growth between its administrations. Similarly, on the tenth day 20 mg/kg of demeclocycline was administered. On day 23, 24 hours after the last injection, the animals were killed by anesthesia with carbon dioxide.

The body weight was recorded throughout the experiment. At autopsy, 0.1 ml of blood was taken for the determination of blood glucose. Serum was prepared from the rest of the blood, and total serum IGF-I levels were determined by RIA. Gastrocnemius muscle, periuterine fat and uterus were removed, dissected free of connective tissue, and weighed.

The results of this experiment are detailed in Tables 1 and 2 and summarized below.

When ovariectomized ("Ovx" in the tables) control animals were compared to sham operated control animals, no significant differences were observed in serum IGF-I levels or in daily body weight gain (Tables 1 and 2). Gastrocnemius muscle mass was increased-by 22% and periuterine fat mass by 48% in ovariectomized controls (Table 2), while trabecular bone weight, calcium and hydroxyproline were substantially reduced (data not shown).

In ovariectomized animals treated with IGF-I/IGFBP-3 complex, the concentration of plasma IGF-I was increased in a dose dependent manner by the three doses of the complex, namely by 14, 32 and 47%, respectively (Table 1). However, due to large variations in the measured values, none of these increases reached statistical significance. This was most likely due to the fact that the samples were taken 24 hours after the last treatment, at which time expected early increases in circulating IGF-I apparently dissipated.

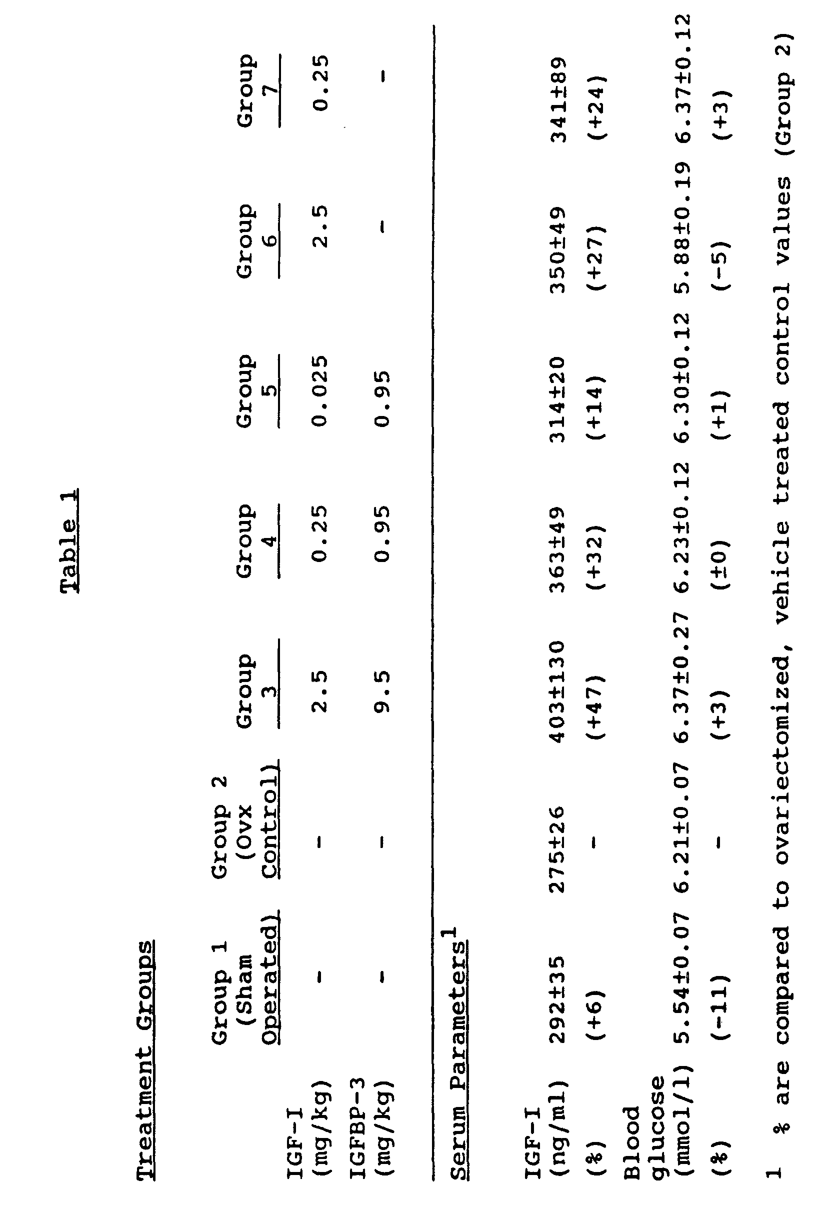

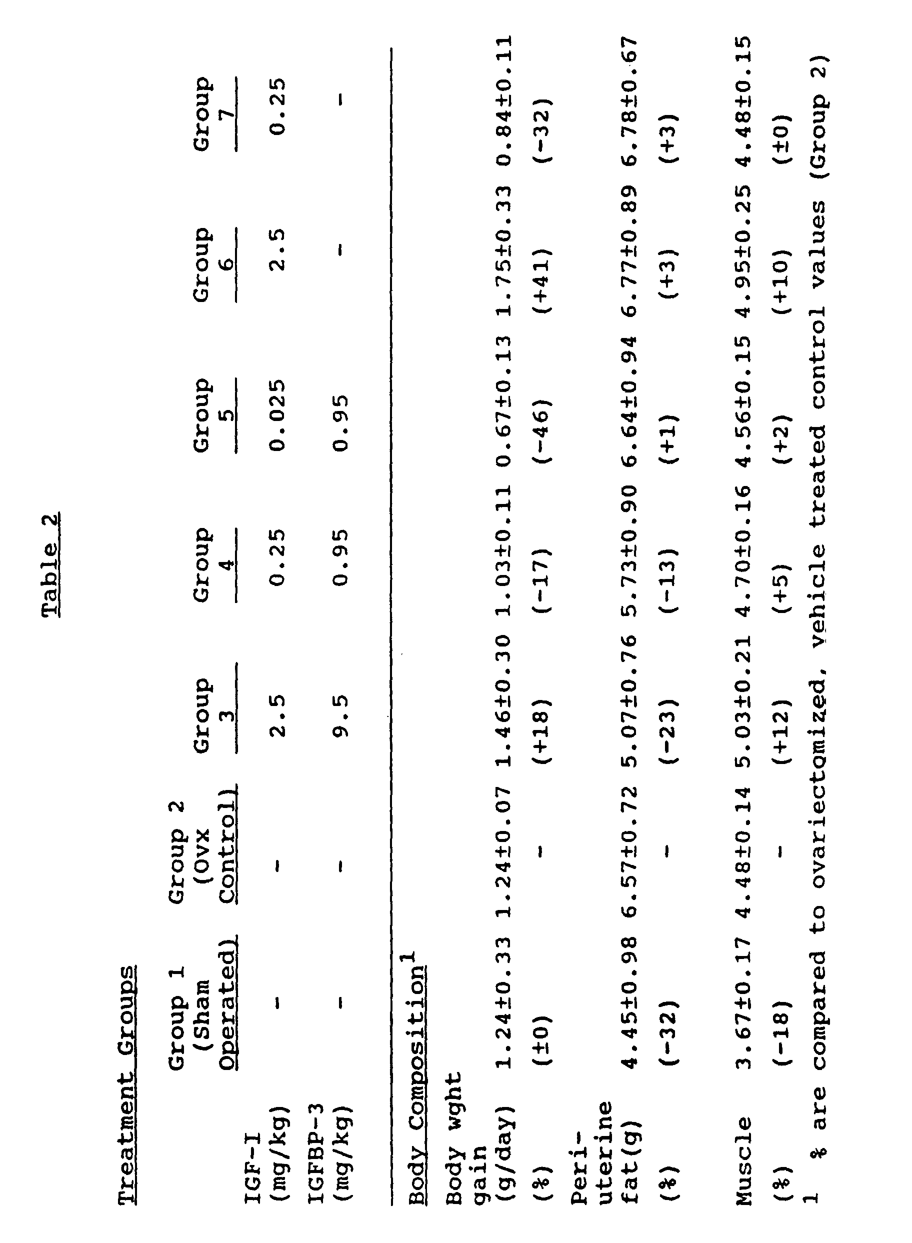

In the rats treated with IGF-I/IGFBP-3 complex, body weight gain was affected biphasically (Table 2). In the group receiving the lowest dose of IGF-I/IGFBP-3 complex, weight gain was reduced by 46%; but in the groups receiving the medium and highest doses, there was no statistically significant change in weight gain.

The trends toward a dose-dependent increase in the weight of the gastrocnemius muscle and a dose-dependent decrease in the weight of periuterine fat with increasing IGF-I/IGFBP-3 complex were apparent (Table 2). At the highest complex dose, muscle mass was increased by 12% above the muscle mass increase observed as a result of ovariectomy alone. Conversely, at the highest complex dose, periuterine fat mass was decreased by 23%, substantially reversing the increase in fat mass resulting from ovariectomy.

Free IGF-I at a dose of 2.5 mg/kg also increased muscle mass, but the increase was not statistically significant; and essentially no effect was seen at a dose of 0.25 mg/kg. Furthermore, neither dose of free IGF-I had an effect on periuterine fat mass.

The combination of the increase in muscle, decrease in fat, and overall maintenance of total body weight indicates the potent effects of the IGF-I/IGFBP-3 complex in promoting muscle accretion and lean body mass development, and demonstrates the effectiveness of the complex.

### Example 3

The effect of a systemically administered complex of IGF and IGFBP was tested on post-surgical healing of animals given the glucocorticoid methylprednisolone.

Sprague-Dawley rats weighing at least 350 grams were selected. On the back of each, four Hunt-Schilling wire mesh wound cylinders were implanted subcutaneously. Each animal was injected subcutaneously with 8 mg of methylprednisolone at the time of surgery. In addition, some rats were given daily subcutaneous injections at a site distant from the implanted wound cylinders of IGF-I or the complex of IGF-1 and IGFBP-3 (both supplied by Celtrix Pharmaceuticals, Santa Clara CA) in PBS and 0.1% rat serum albumin, pH 6.0. The treatment groups were

| | |
|---|---|
| Vehicle | (negative control) |
| IGF-I | 1.25 mg IGF-I/kg/day |
| Complex | 1.25 mg IGF-I/kg/day complexed with an equimolar amount of IGFBP-3 |

On postoperative day 17, the tissue in the wound cylinders was harvested and dried at 37° C. Dry weights, DNA, total protein and hydroxyproline (collagen) contents were obtain according to published procedures. Burton, Biochem. J. (1956) 17: 428-430; Grant, J. Clin. Pathol. (1964) 17: 685-686; and Moore and Stein, J. Biol. Chem. (1954) 211: 907-913.

When values from rats treated with IGF-I were compared with those of the negative control, wound cylinder dry weight increased 250%, DNA increased 340%, total protein increased 200% and hydroxyproline increased 205%. When the results in the animals treated with the complex of IGF-I and IGFBP-3 were compared with those of the negative control, wound cylinder dry weight increased 360%, DNA increased 450%, total protein increased 320% and hydroxyproline increased 250%. All values were significantly higher for the animals administered the complex of IGF-I and IGFBP-3 than for the IGF-I-treated animals.

This experiment indicates that the wound healing defect induced by glucocorticoid administration was reversed by the systemic administration of the complex of IGF-I and IGFBP-3.

### Example 4

The effect of a systemically administered complex of IGF and IGFBP is tested on post-surgical healing of animals given skin wounds.

Young pigs weighing about 10 to 15 kg are fasted for at least six hours before surgery. Anesthesia is administered intravenously, along with a dose of vehicle, IGF-I alone or the complex of IGF-I and IGFBP-3. Under aseptic conditions, the pigs' backs and stomachs are clipped, shaved and washed. Then the area to be wounded is disinfected with 70% alcohol. Next a block of pig skin measuring 1 cm x 1.5 cm x 0.7 cm deep is removed. This depth involves all the epithelium and part of the dermis and is similar in depth to a second degree burn. The wounds are then dressed with sterile dressings, which are replaced daily. The animals receive daily subcutaneous doses of vehicle, IGF-I alone or the complex of IGF-I and IGFBP-3 for five days. On the seventh day, the healing wound is biopsied.

Histologic specimens are prepared using standard paraffin impregnating and embedding techniques. Four micron sections are made and stained using hematoxylin and eosin. Slides are marked with numbers which in a separate book are related to the test and control animals. Other investigators who did not prepare or number the slides measure the widths of epithelial and connective tissue layers under the microscope.

Wounds from animals treated with the complex of IGF-I and IGFBP-3 show the most improvement, followed by those of animals treated with IGF-I. The animals given the vehicle heal at the normal rate.

## Claims

1. The use of a complex comprising an insulin-like growth factor (IGF) and insulin-like growth factor binding protein-3 (IGFBP-3) in the manufacture of a systemically administered medicament, other than one adapted for subcutaneous bolus administration, for treating a catabolic condition.

2. The use according to claim 1 wherein the complex comprises equimolar amounts of IGF and IGFBP-3.

3. The use according to claim 1 or 2 wherein the IGF is IGF-1, such as recombinant human IGF-I, or IGF-II, such as recombinant human IGF-II.

4. The use according to any of claims 1 to 3 wherein the IGFBP-3 is recombinant human IGFBP-3.

5. The use according to any preceding claim wherein the catabolic condition is a protein wasting disease.

6. The use according to any preceding claim wherein the amount of complex administered results in a positive nitrogen balance in the individual.

7. The use according to any preceding claim wherein the amount of amount of complex administered is at least about 0.05 mg of IGF/kg of body weight/day.

8. The use according to any preceding claim wherein the individual treated is a mammalian or avian individual such as a human, cow, hog, sheep, chicken, turkey, duck, goose, dog, horse, cat or pet bird.

9. The use of a composition comprising IGF and IGFBP in the manufacture of a systemically administered medicament, other than one adapted for subcutaneous bolus administration, for:
(a) enhancing repair of soft and hard tissue in an individual about to undergo, undergoing or just having undergone surgery;
(b) treating a burn;
(c) treating an injury by trauma; and/or
(d) treating a peptic ulcer.

10. The use according to claim 9 wherein the individual undergoing surgery is having a tumor resected, is having an organ removed or replaced, is receiving glucocorticoid therapy or is diabetic.

11. The use according to claim 9 or 10 wherein the composition is administered by parenteral, gastrointestinal or nasal administration.

12. The use according to any of claims 9 to 11 wherein the composition is parenterally administered such as by intravenous, intraperitoneal or intramuscular injection.

13. The use according to any of claims 9 to 12 wherein the IGF is IGF-I, such as recombinant human IGF-I and/or the IGFBP is IGFBP-3, such as recombinant human IGFBP-3.

14. The use according to claim 13 wherein the recombinant human IGFBP-3 is non-glycosylated.

15. The use according to any of claims 9 to 14 wherein the IGF is administered at at least about 0.1 - 5 mg of IGF/kg of body weight/day.

16. The use according to any of claims 9 to 15 wherein IGF is complexed to an approximately equimolar amount of IGFBP and/or the individual to be treated is a mammal or bird.

## Patentansprüche

1. Verwendung eines komplexes, umfassend einen Insulin-ähnlichen Wachstumsfaktor (IGF) und das den Insulin-ähnlichen Wachstumsfaktor bindende Protein-3 (IGFBP-3) zur Herstellung eines systemisch verabreichten Medikaments, das anders ist als ein für subkutane Bolusverabreichung angepaßtes Medikament, zur Behandlung eines katabolen Zustands.

2. Verwendung nach Anspruch 1, wobei der Komplex äquimolare Mengen von IGF und IGFBP-3 umfaßt.

3. Verwendung nach Anspruch nach 1 oder 2, wobei der IGF IGF-1 ist, wie rekombinanter menschlicher IGF-I, oder IGF-II, wie rekombinanter menschlicher IGF-II.

4. Verwendung nach einem der Ansprüche 1 bis 3, wobei das IGFBP-3 rekombinantes menschliches IGFBP-3 ist.

5. Verwendung nach einem der vorhergehenden Ansprüche, wobei der katabole Zustand eine Protein-zehrende Erkrankung ist.

6. Verwendung nach einem der vorhergehenden Ansprüche, wobei die Menge des verabreichten Komplexes im Individuum zu einer positiven Stickstoffbilanz führt.

7. Verwendung nach einem der vorhergehenden Ansprüche, wobei die Menge des verabreichten Komplexes mindestens 0.05 mg IGF/kg Körpergewicht/Tag beträgt.

8. Verwendung nach einem der vorhergehenden Ansprüche, wobei das behandelte Individuum ein Säuger oder Geflügel ist, wie Mensch, Kuh, Schwein, Schaf, Huhn, Pute, Ente, Gans, Hund, Pferd, Katze oder ein als Haustier gehaltener Vogel.

9. Verwendung einer Zusammensetzung, umfassend IGF und IGFBP zur Herstellung eines systemisch verabreichten Medikaments, das anders ist als ein für Bolusverabreichung angepaßtes Medikament, für:
(a) Verbesserung der Reparatur von weichem und harten Gewebe in einem Individuum vor, während oder nach einem chirurgischen Eingriff;
(b) Behandlung von Verbrennung;
(c) Behandlung von Verletzung durch Trauma; und/oder
(d) Behandlung eines Ulcus pepticum.

10. Verwendung nach Anspruch 9, wobei bei dem sich einem chirurgischen Eingriff unterziehenden Individuum eine Tumorresektion, ein(e) Organentfernung oder ersatz oder eine Glucocorticoidbehandlurg erfolgt oder es diabetisch ist.

11. Verwendung nach Anspruch 9 oder 10, wobei die Zusammensetzung parenteral, gastrointestinal oder nasal verabreicht wird.

12. Verwendung nach einem der Ansprüche 9 bis 11, wobei die Verbindung parenteral verabreicht wird, wie durch intravenöse, intraperitoneale oder intramuskuläre Injektion.

13. Verwendung nach einem der Ansprüche 9 bis 12, wobei der IGF IGF-I ist, wie rekombinanter menschlicher IGF-I, und/oder das IGFBP IGFBP-3 ist, wie rekombinantes menschliches IGFBP-3.

14. Verwendung nach Anspruch 13, wobei das rekombinante menschliche IGFBP-3 nicht glycosyliert ist.

15. Verwendung nach einem der Ansprüche 9 bis 14, wobei der IGF in einer Menge von mindestens etwa 0.1 bis 5 mg IGF/kg Körpergewicht/Tag verabreicht wird.

16. Verwendung nach einem der Ansprüche 9 bis 15, wobei IGF mit einer ungefähr äquimolarer Menge von IGFBP komplexiert ist und/oder das zu behandelnde Individuum ein Säuger oder Vogel ist.

## Revendications

1. Utilisation d'un complexe comprenant un facteur de croissance analogue à l'insuline (IGF) et une protéine de liaison au facteur de croissance analogue à l'insuline 3 (IGFBP-3) dans la fabrication d'un médicament administré par voie systémique, autre qu'un médicament adapté à une administration en bolus sous-cutanée, pour le traitement d'un état catabolique.

2. Utilisation selon la revendication 1, dans laquelle le complexe comprend des quantités équimolaires d'IGF et d'IGFBP-3.

3. Utilisation selon la revendication 1 ou 2, dans laquelle l'IGF est un IGF-I, tel qu'un IGF-I humain recombiné, ou un IGF-II, tel qu'un IGF-II humain recombiné.

4. Utilisation selon l'une quelconque des revendications 1 à 3, dans laquelle l'IGFBP-3 est une IGFBP-3 humaine recombinée.

5. Utilisation selon l'une quelconque des revendications précédentes dans laquelle l'état catabolique est une maladie à perte protéique.

6. Utilisation selon l'une quelconque des revendications précédentes, dans laquelle la quantité de complexe administré permet d'obtenir un bilan azoté positif chez l'individu.

7. Utilisation selon l'une quelconque des revendications précédentes, dans laquelle la quantité de complexe administré est d'au moins environ 0,05 mg d'IGF/kg de poids corporel/jour.

8. Utilisation selon l'une quelconque des revendications précédentes, dans laquelle l'individu traité est un individu mammifère ou aviaire tel qu'un humain, une vache, un porc, un mouton, un poulet, une dinde, un canard, une oie, un chien, un cheval, un chat ou un oiseau familier.

9. Utilisation d'une composition comprenant un IGF et une IGFBP dans la fabrication d'un médicament administré par voie systémique, autre qu'un médicament adapté à une administration en bolus sous-cutanée, pour :
(a) activer la réparation de tissus mous et durs chez un individu sur le point de subir, subissant, ou venant de subir une intervention chirurgicale ;
(b) traiter une brûlure ;
(c) traiter une lésion par trauma ; et/ou
(d) traiter un ulcère gastro-duodénal.

10. Utilisation selon la revendication 9, dans laquelle l'individu subissant une intervention chirurgicale a une tumeur réséquée, a un organe retiré ou remplacé, reçoit un traitement de glucocorticoïde ou est diabétique.

11. Utilisation selon la revendication 9 ou 10, dans laquelle la composition est administrée par administration parentérale, gastro-intestinale ou nasale.

12. Utilisation selon l'une quelconque des revendications 9 à 11, dans laquelle la composition est administrée par voie parentérale, telle qu'une injection intraveineuse, intrapéritonéale, ou intramusculaire.

13. Utilisation selon l'une quelconque des revendications 9 à 12, dans laquelle l'IGF est un IGF-I, tel qu'un IGF-I humain recombiné, et/ou l'IGFBP est une IGFBP-3, telle qu'une IGFBP-3 recombinée.

14. Utilisation selon la revendication 13, dans laquelle l'IGFBP-3 humaine recombinée n'est pas glycosylée.

15. Utilisation selon l'une quelconque des revendications 9 à 14, dans laquelle l'IGF est administré à au moins environ 0,1 - 5 mg d'IGF/kg de poids corporel/jour.

16. Utilisation selon l'une quelconque des revendications 9 à 15, dans laquelle l'IGF est complexé à une quantité environ équimolaire d'IGFBP et/ou l'individu à traiter est un mammifère ou un oiseau.
